# EUROPÄISCHE PATENTSCHRIFT

(11) **EP 0 585 253 B1**
(45) Veröffentlichungstag und Bekanntmachung des Hinweises auf die Patenterteilung: **10.04.1996**
(21) Anmeldenummer: 92909190.8
(22) Anmeldetag: 24.04.1992
(51) Int. Cl.: A61D 9/00, A61L 15/00, A61F 5/02

(54) **VERWENDUNG EINES GEWEBES ZUR HERSTELLUNG EINES THERAPEUTISCHEN HILFSMITTELS**
USE OF A WOVEN CLOTH FOR PRODUCING THERAPEUTIC AIDS
UTILISATION D'UN TISSU POUR FABRIQUER UN SUPPORT THERAPEUTIQUE

(30) Priorität: 25.04.1991 CH 1241/91
(43) Veröffentlichungstag der Anmeldung: 09.03.1994
(73) Patentinhaber: VIBROSTATIC MARKETING & FINANCE CORPORATION LIMITED, FL-9490 Vaduz (LI)
(72) Erfinder: BENCKHUIJSEN, Gerrit Jan, FL-9492 Eschen (LI)
(74) Vertreter: Kaminski, Susanne, Dr.
(86) Internationale Anmeldenummer: EP9200909
(87) Internationale Veröffentlichungsnummer: WO9219180

(56) Entgegenhaltungen:
- EP-A- 0 059 917
- CH-A- 336 038
- FR-A- 1 231 117
- GB-A- 1 572 250

## Beschreibung

Sauerstoffmangel im menschlichen oder tierischen Gewebe kann als primäre Ursache vieler Beschwerden, Leiden und Krankheiten angesehen werden. Ein Teilgebiet der medizinischen Forschung hat deswegen als Zielrichtung, Methoden zu finden bzw. zu verbessern, die ein vermehrtes Angebot an Sauerstoff an das Gewebe ermöglichen.

Bei verschiedenen Indikationen kommen Methoden zur Anwendung, die eine vermehrte Zufuhr von Sauerstoff ermöglichen sollen. So sind beispielsweise die Oxygenierung, die Sauerstoff-Mehrschritt-Therapie, oder auch die Hyperbarmedizin, oder die äußerliche Applikation von aktiviertem, molekularem Sauerstoff, wie er in Tetrachlordecaoxid vorliegt, bekannt. Jede dieser Methoden findet für gewisse Indikationen Befürworter und zeitigt Erfolge, doch ist nicht zu übersehen, daß gerade auf diesem Gebiet die Meinungen sehr weit auseinandergehen. Sicherlich ist einer der Gründe dafür darin zu sehen, daß eine verbesserte Sauerstoffaufnahme auch gewisse Risiken mit sich bringt, die in der immer in einem gewissen Maße stattfindenden Bildung von gefährlichen Sauerstoff-Spezien wie Singulett-Sauerstoff, Hydroxylradikalen, Superoxidradikalen und Peroxid liegen. Außerdem sind manche Anwendungen per se nicht ungefährlich, man denke dabei an die Hyperbarmedizin oder die Injektionen mit sauerstoffangereichertem Eigenblut.

So war es eine Aufgabe der vorliegenden Erfindung, eine unbedenkliche Methode bereitzustellen, die eine vermehrte Aufnahme von Sauerstoff in das menschliche bzw. tierische Gewebe ermöglicht. Dies gelingt durch die Verwendung eines Stoff-Gewebes, das zu wenigstens 50 Gew.% aus Polyvinylchlorid- und/oder Polyamid-Fasern besteht, gegebenenfalls auch Acryl-Fasern, jedenfalls aber weniger als 10% Baumwoll- und/oder Zellwoll-Fasern enthält zur Herstellung eines therapeutischen Hilfsmittels zur Erhöhung des Sauerstoff-Partialdrucks in der tierischen bzw. menschlichen Muskulatur.

Unter "(Stoff-)Gewebe" ist dabei jede Art von textilem Flächengebilde zu verstehen, wobei die Fadenvereinigung der verschiedenen Fasern in Form von Geweben, Kulierware, Kettenwirkware, Geflechten oder auch vliesartigen Gebilden geschehen kann.

Es ist bekannt, daß aus bestimmten Kunststoff-Fasern, insbesondere einer Mischung von Polyvinylchlorid- und Acryl-Fasern, hergestellte Gewebe besonders vorteilhafte physikalisch-chemische Eigenschaften besitzen. So zeichnen sie sich aufgrund der geringen Wärmeleitfähigkeit der Fasern durch ein hohes Isoliervermögen aus. Auch sind die Fasern wasserabstoßend und quellen nicht auf, so daß Flüssigkeit auf der Haut sofort vom Gewebe nach außen geleitet wird. Dazu kommt, daß beim Tragen dieser synthetischen Fasern Triboelektrizität entsteht; es kommt dabei zu einer Tonisierung der Haut und der Hautanhangsgebilde, eine als äußerst angenehm empfundene Eigenschaft eines solchen Faser-Gewebes.

Die CH-A-336 038 offenbart die Verwendung von aus Polyvinylchloridfäden bestehenden Textilstoffen für Kleidungsstücke, da beim Tragen derselben eine elektrostatische Aufladung erzeugt wird. Diese Eigenschaft zusammen mit der guten Reflexionswirkung solcher Kleidungsstücke für Körperwärme macht demnach derartige Kleidungsstücke für die Bekämpfung von schmerzhaften Leiden, wie Rheuma, geeignet.

Diese vorteilhaften, bekannten Eigenschaften des Gewebes haben seine Verwendung beispielsweise bei der Behandlung von Schmerzsyndromen und seinen Einsatz als Gewebe für orthopädische, therapeutische Mieder und Bandagen nahegelegt und erfolgreich gemacht, sei es als Zervikalkragen, Schultergelenksbandage oder Lumbalorthese, oder als Bandagen für die Extremitäten. Genauso hat sich dieses Gewebe in der Veterinärmedizin für Bandagen der empfindlichen, schwellungsanfälligen Sehnen und Gelenke von Pferden bewährt.

Zwar wurde eine Hyperämie der Haut beim Tragen dieses Gewebes nachgewiesen, doch legt diese Tatsache in keiner Weise nahe, wie beispielsweise aus der Zeitschrift für die gesamte Innere Medizin vom 1.5.1985 (Leipzig) zu entnehmen ist, daß damit auch eine Erhöhung des Sauerstoffpartialdrucks in der Muskulatur verbunden ist. Dieser überraschende Effekt wurde erst durch breit angelegte Messungen des Sauerstoffpartialdrucks in der Muskulatur an Bein und Rücken festgestellt.

Die Ergebnisse dieser Untersuchungen, die an Sportlern und Nicht-Sportlern, älteren und jüngeren, gesunden und kranken Personen stattgefunden haben, legen die Verwendung eines derartigen Gewebes, z.B. Vibrostatic ^{(R)} (eingetragenes Warenzeichen der Firma Temova Establishment, Vaduz), zur Herstellung von therapeutischen Hilfsmitteln nahe, wie Bandagen, Unterbekleidung, Handschuhen oder Strümpfen, zur unterstützenden Behandlung bei akuten und chronischen Sauerstoffmangelsyndromen als Folge gestörter Mikrozirkulation. Beispielhafte Indikationen sind demnach Tendinose, Condylopathie, Torticollis, Restless Legs, Sudeck-Syndrom, Morbus Raynaud, Morbus Bürger, diabetische Angiopathie, Neigung zu Muskelkrämpfen, insbesondere der Wadenmuskulatur, und arterielle Verschlußkrankheiten. Bekleidungsstücke für Sportler aus diesem Gewebe empfehlen sich insbesondere als unterstützendes Hilfsmittel während der Aufwärmphase, zur rascheren und effektiveren Mobilisierung der Muskulatur. Auch die Verwendung zur Herstellung von Bandagen bzw. von Strümpfen zur unterstützenden Behandlung von arteriellen Durchblutungsstörungen in den Gliedmaßen bietet sich an, möglicherweise weil der durch das erfindungsgemäße Gewebe erhöhte Sauerstoffpartialdruck den Sauerstoff-Übergang in das Muskelgewebe verbessert. Daher ist auch eine entsprechende Anwendung in der Veterinärmedizin naheliegend, insbesondere für Sportpferde oder auch für Hunde, bei Indikationen wie Spasmi Musculorum, Hernia Cervicalis, Myalgia Dorsi, Tendinose, Tendovaginitis, Tendinitis, Tumor Traumatica, Sonatia Mala Vulneri, Podotrochleose, Sesamoidose, Arthrose, akute und chronische Arthritis, und Periostitis.

Eine Erhöhung des Sauerstoffpartialdrucks in der Muskulatur bedeutet eine Erhöhung der Mikrozirkulation. Diese Erhöhung der Mikrozirkulation in tieferen Gewebeschichten ist medizinisch keine zwingende Folge der Hyperämisierung der Haut bzw. der subkutanen Bereiche.

Die positiven, unerwarteten Auswirkungen im tieferen menschlichen bzw. tierischen Gewebe beim Tragen des für die erfindungsgemäße Verwendung geeigneten Gewebes werden anhand der Untersuchungsergebnisse offensichtlich, wobei die Untersuchungen verschiedene Versuchsreihen umfassen. Deren Ergebnisse werden anhand der Zeichnung beispielhaft diskutiert. Es zeigen:
- Fig.1: das Meßprinzip anhand der Darstellung der Meßsonde;
- Fig.2 bis 8: die Ergebnisse für eine erste Versuchsreihe und
- Fig.9 bis 12: die Einzelergebnisse für eine zweite Versuchsreihe.

Es wurden zwei Versuchsreihen durchgeführt. In der ersten Versuchsreihe wurde der Sauerstoffpartialdruck in der Beinmuskulatur, in der zweiten Versuchsreihe der Sauerstoffpartialdruck in der Lendenwirbelsäulenmuskulatur gemessen. Den Probanden wurde nach einem Randomisierungsschema Wäsche, d.h. Strümpfe bzw. miederartige Leibchen, angelegt. Diese direkt auf der Haut getragenen Unterbekleidungsstücke waren dabei aus unterschiedlichen Fasern hergestellt. So waren sowohl nur aus Kunststoff-Fasern, als auch nur aus Naturfasern hergestellte Bekleidungsstücke im Einsatz, genauso wie Mischungen davon.

Wie aus diesen Untersuchungsserien zu ersehen ist, sind zwei Fasern bzw. Fasermischungen extreme Meßergebnisse zuzuordnen.

Diese beiden Materialien sind einerseits reine Baumwolle (negativ!) und andererseits ein Gewebe aus einer Mischung von ca. 85% Polyvinylchlorid- (PVC-) und 15% Acryl-Fasern, im folgenden der Einfachheit halber als PVC-A-Faser bezeichnet (positiv!). Die Ergebnisse für Bekleidungsstücke aus anderen Fasern bzw. Fasergemischen lagen zwischen den negativen Extremwerten für aus reiner Baumwolle hergestellten Bekleidungsstücken und den positiven Extremwerten für solche aus PVC-A-Fasern. Besonders gute Ergebnisse waren für reine PVC-Fasern, abgestuft dazu auch noch für reine Polyamid-Fasern und Mischungen dieser beiden erreichbar. Die Werte für Seide und Wolle bzw. Mischungen dieser Fasern untereinander zeigen ein weitgehend neutrales Verhalten, so daß Zumischungen dieser Fasern zu den erfindungsgemäßen Gemischen zur Erzielung bestimmter Eigenschaftskombinationen zweckmäßig sein mögen. Die Werte für Angora, die in der folgenden Aufstellung nicht angeführt sind, sind zwar grundsätzlich auch positiv zu sehen. Daß Angorawolle allein sich trotzdem für die Herstellung eines therapeutischen Hilfsmittels nicht so gut eignet, liegt an der gleichzeitig als negativ zu bewertenden Eigenschaft, Feuchtigkeit aufzusaugen. Damit wird, direkt auf der Haut getragen, kein vergleichbar guter Tragekomfort geboten, wie beispielsweise durch ein Bekleidungsstück aus PVC-A-Faser. Mischungen allerdings, die Angora- und/oder PVC-A-Fasern enthalten, können für gewisse Anwendungsfälle von Vorteil sein. Auch Beimischungen von Seide zeigen sich unter dem Gesichtspunkt des Tragekomforts als vorteilhaft.

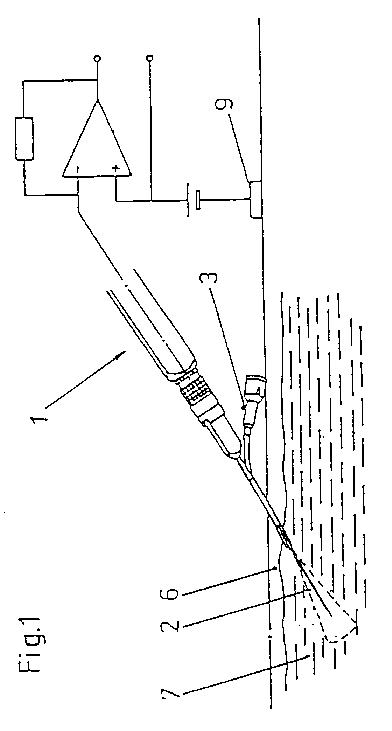

Aus Fig.1 ist das Meßprinzip für die Messungen des Sauerstoffpartialdrucks zu ersehen. Die Messungen erfolgten jeweils vor und eine Stunde nach Tragen der Strümpfe oder des Mieders. Der Sauerstoffdruck wurde mit einem Histographen 1 bestimmt. Dabei wird die Haut 6 mit einer Kanüle 3 durchstochen. Durch diese Kanüle 3 wird eine 0,35 mm dünne Meßsonde 2 aus Gold eingeführt. Diese Sonde 2 wird gegen eine Ag/AgCl-Gegenelektrode 9 mit ca 700 mV vorgespannt. Der daraufhin fließende Strom ist in seiner Stärke dem Sauerstoffpartialdruck im verbindenden Elektrolyten, hier also dem Muskelgewebe des Probanden, proportional. Mittels eines automatischen, programmierten Systems wird die Sonde 2 in die Muskulatur 7 in Schritten vorgeschoben. Die jeweilige Meßzeit ist so kurz, daß durch den mechanischen Druck der Sonde 2 selbst auf die Umgebung keine Störungen des Sauerstoffdruckes in der Muskulatur 7 zu erwarten sind. Es erfolgen 200 Messungen, die in einem bestimmten Muskelareal vorgenommen werden. Hierdurch ist gesichert, daß der errechnete Mittelwert die tatsächliche Situation in der Muskulatur wiedergibt. Vor der Sauerstoffpartialdruckmessung wurde jeweils die Gewebetemperatur mittels eines schnell reagierenden Meßfühlers registriert.

### Versuchsdurchführung:

Versuchsreihe 1 hatte den Vergleich verschiedener Materialien für Strümpfe und Messungen in der Oberschenkelmuskulatur zum Ziel.

Die Probanden waren 16 gesunde Personen, Männer und Frauen im Alter von 20 bis 31 Jahren. Sie wurden gebeten, sich drei Stunden vor der Messung nicht körperlich anzustrengen. Vor dem Versuchsbeginn mußte weitgehend Ruhe eingehalten werden. Die Messungen erfolgten im Liegen. Eine Anaesthesierung der Haut erfolgte nicht.

Nach dem Einstechen der Führungskanüle 3 durch die Haut 6 wurde zunächst die Temperatur im Gewebe gemessen und dann die Sauerstoffpartialdruck-Meßsonde 2 eingeführt. Die gesamte Meßzeit betrug 4 Minuten. Dann wurde auch die Führungskanüle 3 entfernt und der Strumpf - aus den unterschiedlichen Fasern - über das Bein gezogen. Er reichte dann vom Knöchel bis zur Schenkelbeuge. Der Strumpf wurde eine Stunde anbehalten. Der Proband wurde gebeten, während dieser Zeit seine Beinmuskulatur so wenig wie möglich zu belasten.

Versuchsreihe 2 hatte als Ziel, die Wirkung in bezug auf die Erhöhung des Sauerstoffpartialdrucks beim Tragen von miederartigen Leibchen aus PVC-A-Fasern aufzuzeigen.

Die Untersuchungen wurden an acht Personen durchgeführt. Die Messungen erfolgten nach dem gleichen Schema wie in der Versuchsreihe 1. Das Nieder wurde ebenfalls für eine Stunde getragen. Um die sehr störenden Atembewegungen in der Lumbalregion so gering wie möglich zu halten, wurde auf eine bequeme Bauchlagerung Wert gelegt.

### Ergebnisse:

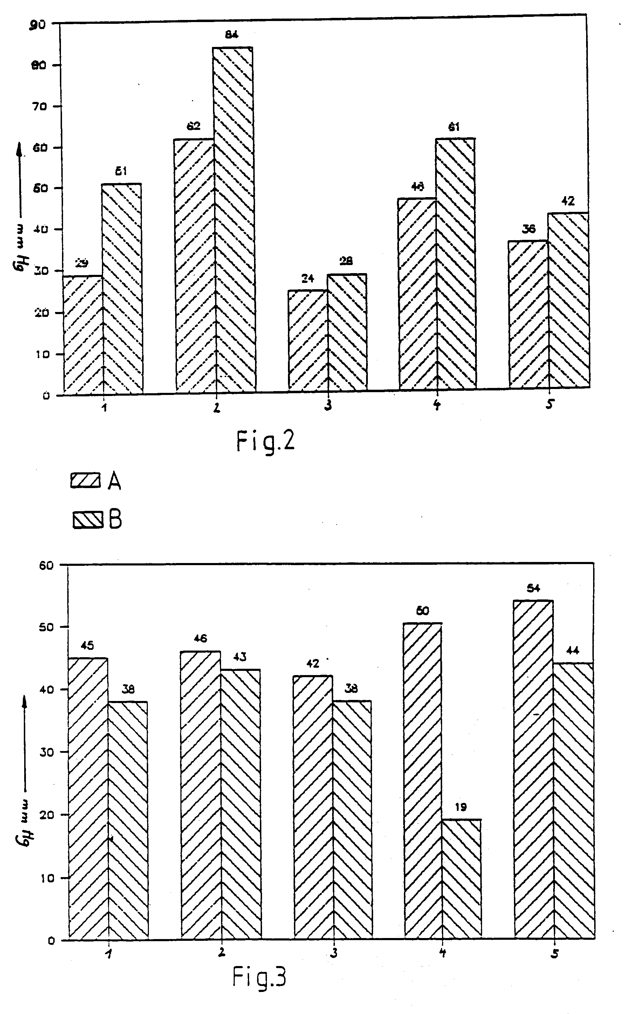

Versuchsreihe 1: Aus den Fig.2 bis 8 sind die Meßergebnisse zu entnehmen. Fig.2 zeigt den Gewebesauerstoff-Partialdruck in mm Hg für Probanden der Gruppe I (PVC-A-Faser-Strümpfe), wobei A die Ausgangswerte und B die Werte nach einer Stunde Tragen darstellen. Fig.3 zeigt die gleichen Werte für die Gruppe II (Baumwoll-Strümpfe).

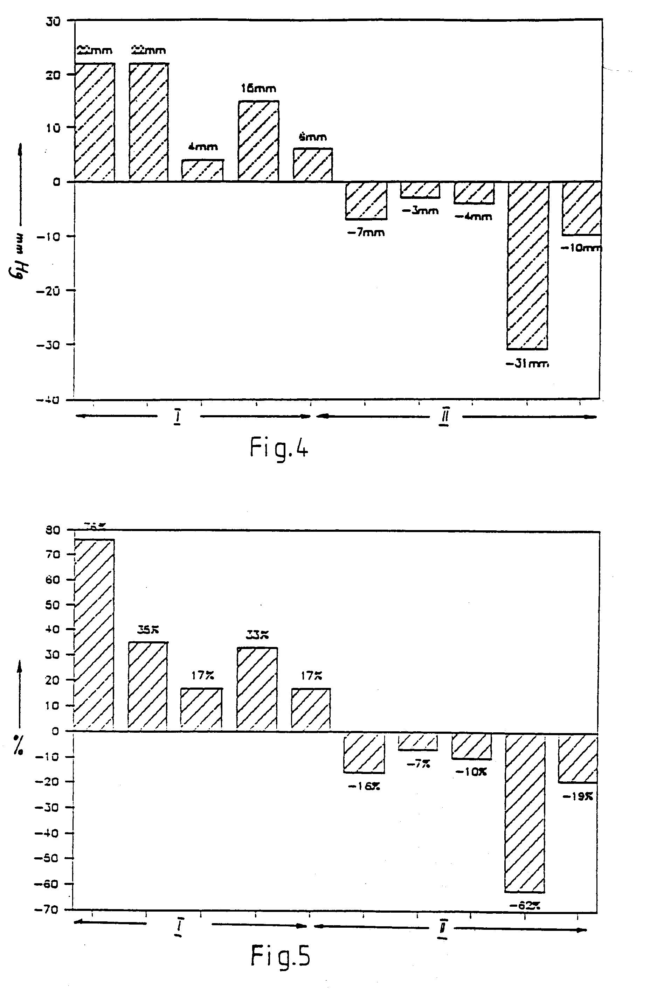

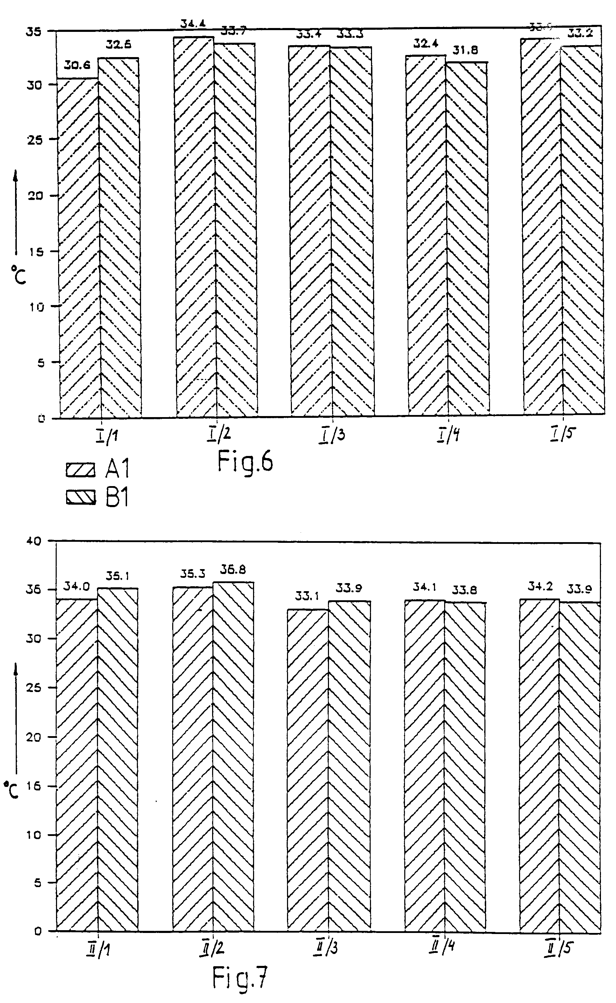

Fig.4 und 5 zeigen die Zu- bzw. Abnahme des Sauerstoffpartialdrucks für beide Gruppen; Fig.4 in mm Hg, Fig.5 in Prozenten.

Bei jeder Versuchsperson der Gruppe I steigt der Sauerstoff-Partialdruck nach einstündigem Tragen des PVC-A-Faser-Strumpfes an. Bei jeder Versuchsperson der Gruppe II fällt der Sauerstoff-Partialdruck nach einstündigem Tragen des Baumwollstrumpfes ab. Die Größe des Abfalls des Sauerstoff-Partialdrucks beim Tragen von Baumwollstrümpfen wird in nicht unbeträchtlicher Weise durch die Art und Menge von Zumischungen bestimmt. So ist der Abfall des Sauerstoff-Partialdrucks für Strümpfe aus reiner Baumwolle am größten; werden beispielsweise elastische Acryl-Kräuselfäden beigemischt, so ist der Abfall - wie entsprechend den Untersuchungen zu erwarten ist - geringer.

Aus Fig.6 und 7 sind die Ausgangswerte A1 und die Werte B1 (nach einstündigem Tragen der Strümpfe) der Gewebetemperatur der Probanden zu entnehmen, wobei Fig.6 die Ergebnisse für die PVC-A-Faser-Strümpfe tragenden Probanden und Fig.7 diejenigen für die Baumwollstrümpfe tragenden Probanden zeigt. Die Temperaturänderungen sind vernachlässigbar klein. Unterscheiden sich auch die Ausgangswerte A1 der beiden Gruppen trotz der Randomisierung, so liegen sie doch im Normbereich, wenn auch für die Gruppe I mehr im unteren und für die Gruppe II im oberen Normbereich. Für die Probanden der Gruppe I lag der Mittelwert der Änderung der Gewebetemperatur bei -0.02°C, für die Probanden der Gruppe II bei -0.56°C.

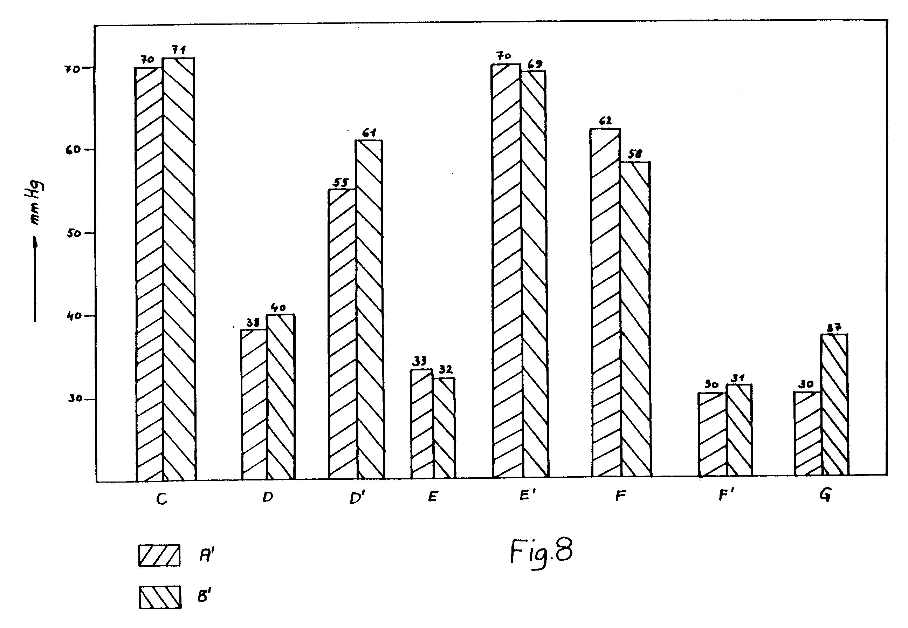

Die Fig.8 zeigt die durchschnittliche Änderung des Sauerstoffpartialdrucks für Probanden beim Tragen von Strümpfen aus 100% Acryl (ein Proband C), 100% Polyamid (zwei Probanden D und D'), 100% Wolle (zwei Probanden E und E'), 100% Seide (zwei Probanden F und F') und 100% PVC (ein Proband G). Auch hier sind die jeweiligen Ausgangswerte A' und die Werte B' (nach einstündigem Tragen der Strümpfe) der Probanden gegen-einander aufgetragen.

### Versuchsreihe 2:

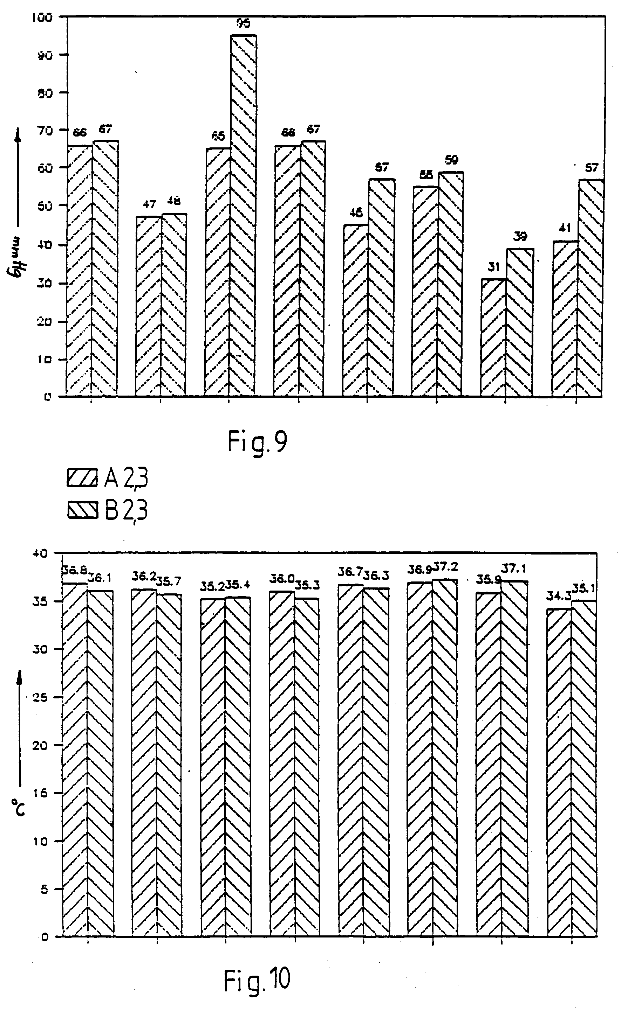

Die Fig.9 bis 12 zeigen die Ergebnisse dieser Versuchsreihe. Der Anstieg des Sauerstoffpartialdrucks ist bei den einzelnen Personen unterschiedlich hoch. Probanden, denen die Lagerung auf dem Bauch keine große Mühe bereitete, und die während der Messung weitgehend auf Bauchatmung verzichten konnten, zeigten in jedem Fall eine Erhöhung des Sauerstoff-Partialdrucks oder zumindest konstante Werte. Jedoch ist zu bedenken, daß Bewegungen der Muskulatur, die zu einer Lageänderung führen, erhebliche Störungen der Meßergebnisse bedingen können. War dies bei der Durchführung der Versuchs reihe 1 unproblematisch, so ergaben sich Schwierigkeiten bei der Versuchsreihe 2, da die auf dem Bauch liegenden Probanden teilweise über Beschwerden klagten.

Fig.9 zeigt die Ausgangswerte A2 und die Werte B2 nach einstündigem Tragen des Mieders für den Sauerstoff-Partialdruck.

Fig.10 zeigt die entsprechenden Werte A3 und B3 der Gewebetemperatur.

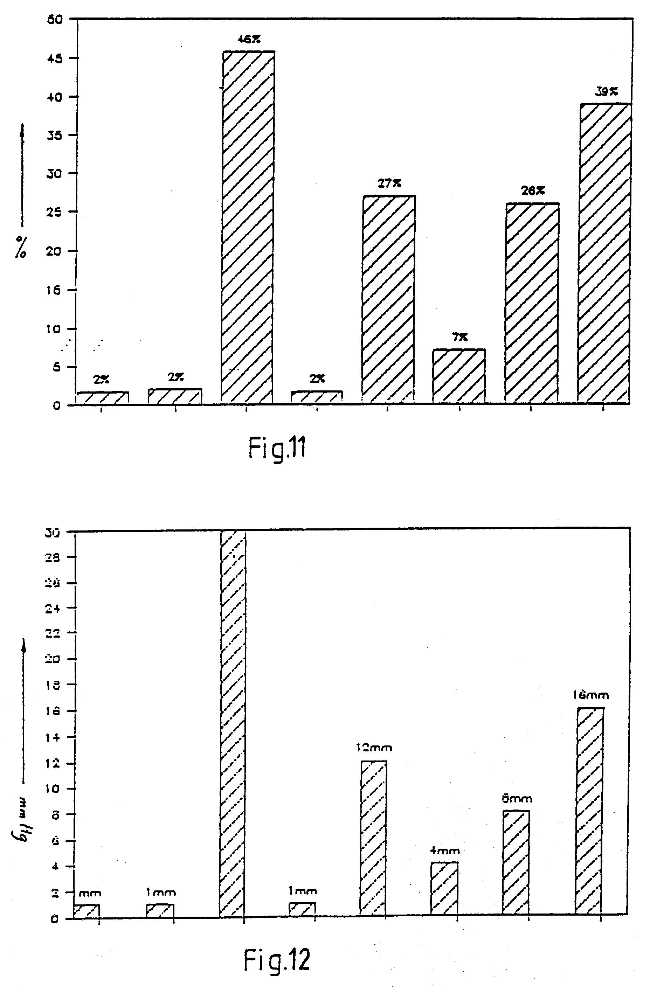

Fig.11 und 12 zeigen die Zunahme des Sauerstoff-Partialdrucks, wobei Fig.11 die Zunahme in % und Fig.12 in mm Hg angibt.

Diskussion der Ergebnisse der beiden Versuchsreihen: Meßproblematik: Die Messung des Sauerstoff-Partialdrucks mittels einer eingeführten Sonde ist, wie schon oben aufgeführt, mit methodischen Schwierigkeiten behaftet. Die Insertion eines Meßkatheters führt zwangsläufig zu Irritationen im Meßbereich des Gewebes, und es kann zu einer deutlichen Beeinflußung der Blutzirkulation und damit der Sauerstoffversorgung kommen. Um diese Probleme zu minimieren, wurde eine sehr feine Sonde von 0,35 mm Durchmesser benutzt. Sie wurde in vorgegebenen Zeitabständen in das Gewebe vor- und zurückgeführt, sowie durch Drehung des Nadelhalters in neue Meßpositionen gebracht. Da die Meßzeit äußerst kurz war, konnten Störungen des Sauerstoff-Partialdrucks durch den Kompressionsdruck der Sonde, wenn überhaupt, erst nach dem Meßvorgang eintreten. Mögliche Fehlmessungen können jedoch zu Lasten der Art des Gewebes gehen. Die Muskulatur mit ihren vielfältigen Strukturen kann durchaus auch außergewöhnliche Meßergebnisse liefern, je nachdem, in welchem Teil des Gewebes vorwiegend gemessen wurde. Durch sorgfältige Auswahl des Meßbereichs wurde jedoch versucht, sicher zu stellen, daß das Mittel von 200 Meßdaten den aktuellen Sauerstoff-Partialdruck wiedergibt. Jedoch war dazu eine ruhende Muskulatur Voraussetzung, da das Meßsystem starr befestigt war und den vorgegebenen Meßbereich abtastete.

Ausgangswerte A: Der Ausgangswert A für den Sauerstoff-Partialdruck lag in dem aufgrund von anderen Messungen erwarteten Bereich. In Abhängigkeit vom trainingsbedingten Myoglobingehalt wurden die höchsten Werte bei Ausdauersportlern festgestellt. Ähnlich wie die Gewebetemperatur fällt der Sauerstoff-Partialdruck mit der Entfernung der Muskulatur von der Körpermitte ab. Je distaler die Muskulatur desto geringer die Gewebetemperatur und der Sauerstoff-Partialdruck in Ruhe. Zum besseren Vergleich der Ergebnisse wurde versucht, die Streuung der Ausgangswerte möglichst gering zu halten, die Probanden demzufolge aufgefordert, vier Stunden vor der Messung keine körperlichen Arbeiten durchzuführen und insbesondere keinen Sport zu treiben. Extremwerte, wie Proband 2 der Gruppe I zeigt (Fig.2) können auf der Tatsache beruhen, daß es sich dabei um einen sehr gut ausdauertrainierten Sportler handelt.

Versuchsreihe 1: Bei allen Probanden der Gruppe I, die Strümpfe aus PVC-A-Fasern getragen haben, kam es nach einstündigem Tragen zu einem Ansteigen des Sauerstoff-Partialdrucks im Oberschenkelmuskel. Bei allen Probanden der Gruppe II, die Baumwollstrümpfe getragen haben, wurde ein Absinken des Sauerstoff-Partialdrucks festgestellt. Eine signifikante Erhöhung des Sauerstoff-Partialdrucks war auch für Probanden festzustellen, die Strümpfe aus reinem Polyvinylchlorid bzw.Polyamid getragen haben.

Versuchsreihe 2: Erwartungsgemäß liegt hier der Ausgangswert für die Gewebetemperatur nahe der Körperkerntemperatur. Für die gemessenen Ausgangswerte des Sauerstoff-Partialdrucks waren keine Vergleichsmessungen bekannt. Obwohl bei Wirbelsäulenfehlstellungen, an denen einige der Probanden litten, durchaus höhere Sauerstoff-Partialdruck-Werte infolge einer starken Beanspruchung der Lendenmuskulatur möglich sind, sinken diese Werte bei ruhiger Lagerung ab. Im allgemeinen wurde auch für diese Versuchsreihe, bei der die Probanden Mieder aus PVC-A-Fasern trugen, eine Erhöhung des Sauerstoff-Partialdrucks in der Lendenmuskulatur gemessen.

## Patentansprüche

1. Verwendung eines Gewebes, das zu wenigstens 50 Gew.% aus Polyvinylchlorid- und/oder Polyamid-Fasern besteht, gegebenenfalls auch Acryl-Fasern, jedenfalls aber weniger als 10 Gew.% Baumwoll- und/oder Zellwoll-Fasern enthält, zur Herstellung eines therapeutischen Hilfsmittels zur Erhöhung des Sauerstoff-Partialdrucks in der tierischen bzw. menschlichen Muskulatur.

2. Verwendung nach Anspruch 1, dadurch gekennzeichnet, daß das Gewebe aus wenigstens 50 Gew.% Polyvinylchlorid-Fasern, vorzugsweise aus mehr als 75 Gew.% Polyvinylchlorid- und maximal 25 Gew.% Acryl-Fasern, besteht.

3. Verwendung nach Anspruch 1 oder 2, dadurch gekennzeichnet, daß das Gewebe zusätzlich Woll-, insbesondere Angora- und/oder Seiden-Fasern enthält.

4. Verwendung nach einem der vorhergehenden Ansprüche zur Herstellung von Wäsche für Sportler, insbesondere für Leistungssportler, zur Erhöhung des Sauerstoff-Partialdrucks in der Muskulatur, vorzugsweise als unterstützendes Hilfsmittel für die Aufwärmphase.

5. Verwendung nach einem der Ansprüche 1 bis 3 zur Herstellung von Bandagen oder Strümpfen zur unterstützenden Behandlung von arteriellen Durchblutungsstörungen in den Gliedmaßen.

6. Verwendung nach einem der Ansprüche 1 bis 3, zur Herstellung eines therapeutischen Hilfsmittels zur Erhöhung des Sauerstoff-Partialdrucks in der Pferde-Muskulatur.

7. Verwendung nach Anspruch 6, zur Herstellung von Bandagen oder Decken für Pferde, insbesondere für Renn-, Dressur- oder Springpferde, vorzugsweise als unterstützendes Hilfsmittel für die Aufwärmphase.

8. Verwendung nach einem der Ansprüche 1 bis 3, zur Herstellung eines therapeutischen Hilfsmittels zur Erhöhung des Sauerstoff-Partialdrucks in der Hunde-Muskulatur.

9. Verwendung nach Anspruch 8 zur Herstellung von Bandagen oder Decken für Hunde.

## Claims

1. The use of a woven cloth, composed of at least 50% by weight of polyvinyl chloride and/or polyamide fibres, that also if necessary contains acrylic fibres, in any case however less than 10% by weight of cotton and/or rayon fibres, for producing a therapeutic aid for increasing the oxygen partial pressure in animal or human musculature.

2. The use as claimed in Claim 1, characterized in that, the cloth is composed of at least 50% by weight of polyvinyl chloride fibres, preferably more than 75% by weight of polyvinyl chloride and not more than 25% by weight of acrylic fibres.

3. The use as claimed in Claim 1 or 2, characterized in that, the cloth additionally contains wool fibres, more in particular angora and/or silk fibres.

4. The use as claimed in any one of the preceding Claims for producing underwear for sportsmen, more in particular for top-performance sportsmen, for increasing the oxygen partial pressure in the musculature, preferably as an auxiliary aid for the warming-up stage,

5. The use as claimed in any one of Claims 1 to 3 for producing bandages or stockings as an aid in the treatment of disturbances in the arterial blood circulation in the limbs.

6. The use as claimed in any one of Claims 1 to 3, for producing a therapeutic aid for increasing the oxygen partial pressure in the musculature of horses.

7. The use as claimed in Claim 6, for producing bandages or horse-cloths, more specifically for race, dressage or jumping horses, preferably as an auxiliary aid for the warming-up stage.

8. The use as claimed in any one of Claims 1 to 3, for producing a therapeutic aid for increasing the oxygen partial pressure in the musculature of dogs.

9. The use as claimed in Claim 8, for producing bandages or cover cloths for dogs.

## Revendications

1. Utilisation d'un tissu constitué d'un mélange d'au moins 50% en masse de fibres de chlorure de polyvinyle et/ou de polyamides, éventuellement également de fibres acryliques, mais en tout état de cause de moins de 10% de coton et/ou de fibranne pour la réalisation d'un moyen thérapeutique destiné à augmenter la pression d'oxygène partielle dans la musculature des animaux ou de l'homme.

2. Utilisation selon revendication 1, caractérisée en ce que le tissu est constitué d'au moins 50% en masse de fibres de chlorure de polyvinyle, et de préférence de plus de 75% en masse de fibres de chlorure de polyvinyle et de 25% en masse au maximum de fibres acryliques.

3. Utilisation selon revendication 1 ou 2, caractérisée en ce que le tissu contient en outre des fibres de laine, notamment de laine angora et/ou des fibres de soie.

4. Utilisation selon l'une des revendications 1 à 3 pour la réalisation de vêtements pour sportifs, notamment pour sportifs de haut niveau, dans le but d'augmenter la pression partielle d'oxygène dans la musculature, de préférence sous la forme de vêtements de contention portés lors de la phase d'échauffement.

5. Utilisation selon l'une des revendications 1 à 3 pour la réalisation de bandages ou de bas de contention dans le but d'assister le traitement de la perturbation de la circulation artérielle dans les membres.

6. Utilisation selon l'une des revendications 1 à 3 pour la réalisation d'un moyen thérapeutique destiné à augmenter la pression partielle d'oxygène dans la musculature des chevaux.

7. Utilisation selon la revendication 6 pour la réalisation de bandages ou de couverture pour chevaux, notamment pour chevaux de course, de dressage ou de saut, de préférence sous la forme de vêtements de contention portés lors de la phase d'échauffement.

8. Utilisation selon l'une des revendications 1 à 3 pour la réalisation d'un moyen thérapeutique destiné à augmenter la pression partielle d'oxygène dans la musculature des chiens.

9. Utilisation selon la revendication 6 pour la réalisation de bandages ou de couverture pour chiens.
